# EUROPÄISCHE PATENTANMELDUNG

(11) **EP 2 156 841 A1**
(43) Veröffentlichungstag der Anmeldung: **24.02.2010**
(21) Anmeldenummer: 09013177.2
(22) Anmeldetag: 02.02.2007
(51) Int. Cl.: A61K 35/14, A61P 37/00

(54) **Konditionierte Blutzusammensetzung und Verfahren zu deren Herstellung**

(30) Priorität: 03.02.2006 DE 102006005016
(62) Teilanmeldung aus: 07711428.8
(71) Anmelder: Orthogen AG, 40210 Düsseldorf (DE)
(72) Erfinder: Reinecke, Julio, Dr., 50733 Köln (DE); Wehling, Peter, Prof. Dr., 40597 Düsseldorf (DE)
(74) Vertreter: Von Renesse, Dorothea

(57) **Zusammenfassung**

Die vorliegende Erfindung betrifft Verfahren zur Herstellung konditionierter Blutzusammenseizungen, die induzierte Faktoren und Cytokine, insbesondere Interleukin-6, enthalten, sowie konditionierte Blutzusammensetzungen, herstellbar durch das Verfahren, und deren Verwendung zur Behandlung oder Vorbeugung eines Leidens des menschlichen oder tierischen Körpers.

## Beschreibung

Die vorliegende Erfindung betrifft Verfahren zur Herstellung konditionierter Blutzusammensetzungen, die induzierte Faktoren oder Cytokine enthalten, sowie konditionierte Blutzusammensetzungen und deren Verwendung zur Behandlung oder Vorbeugung eines Leidens des menschlichen oder tierischen Körpers.

### Stand der Technik

Es ist bekannt, dass Blutkomponenten, vor allem Proteine, Faktoren oder Cytokine wie Erythropoietin, Insulin oder Interferon, die im Blut beziehungsweise Blutserum enthalten sind, therapeutisch oder prophylaktisch wirksam sind. Bekannte Faktoren wie der Interleukin-1-Rezeptorantagonist (IL-1Ra) hemmen die Wirkung entzündungsauslösender Prozesse. Es ist weiter bekannt, dass solche Blutkomponenten teilweise vom Blutgewebe selbst produziert beziehungsweise aus den Blutzellen in die Plasmaphase des Blutes sezemiert werden.

Die Produktion oder Freisetzung bestimmter Blutkomponenten wie Faktoren oder Cytokine kann beispielsweise durch Inkubation von aus einem tierischen oder menschlichen Körper entnommenen Vollblut gesteigert werden. Nach Inkubation liegt dann oft eine höhere Konzentration bestimmter Faktoren im inkubierten Blut vor. Diese Blutkomponenten können dann gegebenenfalls isoliert werden. Auch kann das die induzierten Faktoren enthaltende Blut von zellulären Bestandteilen befreit und als so genanntes induziertes Blutserum in den tierischen oder menschlichen Körper (re)appliziert werden.

Der Vorgang der Steigerung der Produktion oder Freisetzung von Blutkomponenten wie Faktoren oder Cytokine wird als "Induktion" bezeichnet. Ein bekanntes Verfahren zur Induktion von Blutkomponenten in Vollblut besteht im Wesentlichen darin, Vollblut einem tierischen oder menschlichen Körper zu entnehmen und dann in einer modifizierten Einmalspritze, worin spezielle mit Chromschwefelsäure behandelte Glaskugeln vorliegen, für eine bestimmte Zeit unter Kultivierungsbedingungen zu inkubieren (Meijer et al. Inflamm. res. 52 (2003):1-4). Anschließend werden die zellulären Bestandteile abgetrennt, sodass eine konditionierte Blutserumzusammensetzung erhalten wird, worin einige Faktoren oder Cytokine induziert sind. Demgemäß wird aus menschlichem venösern Vollblut ein Serum gewonnen, worin der Anteil der anti-inflammatorischen Cytokine Interleukin-1-Rezeptorantagonist (IL-1Ra), Interleukin-4 (IL-4) und Interleukin-10 (IL-10) gegenüber frisch entnommenem Vollblut erhöht ist. Die Inkubationsdauer des Vollbluts beträgt dabei 24 Stunden. die Inkubationstemperatur etwa 37 °C.

Derart hergestellte Blutserumzusammensetzungen werden eingesetzt zur Behandlung verschiedener entzündlicher Erkrankungen und Autoimmunkrankheiten beispielsweise die rheumatoide Arthritis. Es zeigt sich, dass beispielsweise die rheumatoide Arthritis mittels lokaler und/oder systemischer Verabreichung solcher konditionierter Blutserumzusammensetzungen gelindert oder geheilt werden kann. Die Effektivität der Therapie ist jedoch verbesserungswürdig.

Darüber hinaus gibt es weitere Erkrankungen, beispielsweise Muskelverletzungen, die zumindest im Tiermodel durch lokale beziehungsweise systemische Verabreichung von gentechnisch hergestellten Cytokinen wie IL-1Ra und ähnlichen therapiert werden können. Die auf bekannte Weise herstellbaren Blutserumzusammensetzungen zeigen hier keine oder nur unzureichende Effekte. Dabei sind Muskelverletzungen gerade im Bereich der Sportmedizin häufig; sie haben einen Anteil von bis zu 30 % an den durch Sport erworbenen Krankheiten oder Verletzungen. Über 90 % dieser Muskelverletzungen werden entweder durch Quetschung oder durch extreme Dehnungsbelastung des Muskels verursacht. Regelmäßig führen diese Verletzungen zu starken Schmerzen und in Folge zur Unfähigkeit, weiter zu trainieren beziehungsweise den Sport kurzfristig oder dauerhaft weiter auszuüben. Der Stand der Technik ist deshalb verbesserungswürdig.

Eine emstzunehmende Erkrankung beim Pferd stellt die chronische oder periodische Augenentzündung (equine rezidivierende Uveitis. ERU) dar. Nach dem heutigen Stand der Technik geht man bei dieser chronischen Entzündung davon aus, dass die Allergie gegen sogenannte Leptospiren sowie eine akute oder chronische Leptospireninfektion eine Bedeutung für die Entstehung der chronischen Augenentzündung hat. In Deutschland herrscht ein Durchseuchungsgrad von bis zu 80 % für diese parasitären Organismen. Bei einem Teil infizierter Tiere manifestieren sich verschiedene Krankheitsbilder, darunter auch die chronische Augenentzündung. Entscheidend für die Krankheitsentstehung scheint dabei zu sein, ob das Immunsystem des Tieres die Leptospiren als parasitäre Organismen toleriert oder nicht.

Bei Pferden kann Lahmheit ausgehend von einer massiven Entzündung beziehungsweise Reizung der Sehnenscheide auftreten. Eine weitere Ursache der Lahmheit können auch degenerative Veränderungen innerhalb des Sehnengewebes darstellen, sog. "Core Lesions" Diese ziehen ebenfalls massive Entzündungsreaktionen nach sich. Die Symptome von Entzündungen und Lahmheit werden in der Regel mit Glucocorticoiden (Cortison), Zellmazeraten (ACell®), Thrombozytenkonzentraten (Osteokin®, Magellan® etc.), oder aber Zellpräparationen aus Knochenmark oder Fettgewebe ("Stammzellen") behandelt.

Das Krankheitsbild der Neurodermitis wird durch eine Überreaktion des Immunsystems hervorgerufen. Eine derzeit häufig angewandte Therapie mit cortisonhaltigen Salben ist jedoch mit einigen Nebenwirkungen behaftet.

Weiterhin treten in der Bevölkerung häufig Entzündungen, beziehungsweise Reizungen Irritationen des Nervensystems mit meist unbekannter Genese auf. Dabei stellen Rückenschmerzen eine häufig auftretend Symptomatik dar. Durch Schmerzen als Ursache einer Entzündung können dabei häufig nur symptomatisch durch die Gabe von Schmerzmitteln, beziehungsweise durch Glucocorticoide (wie Triamcinolon) behandelt werden.

Endometriose ist eine Erkrankung, bei der sich Zellen beziehungsweise Gewebe aus der Uterusschleimhaut in der Bauchhöhle ansiedeln und dort zu meist gutartigen Geschwulsten führen. Diese Geschwulst ist meist hormonempfindlich und erzeugt, je nach Hormonlage starke Schmerzen. Die operative Resektion oder eine Hormonbehandlung können gegen die mit dieser Krankheit assoziierten Beschwerden Abhilfe schaffen. Jedoch kommt es regelmäßig zu Rückfällen und Rezidiven. Geschwulste können sich soweit chronifizieren, dass weitere Organe verwachsen und starke chronische Schmerzen entstehen. Die Symptomatik kann oftmals nur mit starken Schmerzmitteln erträglich gemacht werden. Etwa 10 % aller Frauen zwischen Pubertät und Menopause erkranken an Endometriose und entwickeln mehr oder minder starke Symptome. Eine extreme Ausprägung dieser Erkrankung kann zu Unfruchtbarkeit führen.

Es besteht daher Bedarf an verbesserten, alternativen und einfach herstellbaren Wirkstoffzusammensetzungen und Verfahren zu deren Herstellung für die effektive Behandlung der vorbezeichneten Erkrankungen sowie weiteren Erkrankungen, die mittels im Blut vorkommender Faktoren oder Cytokine behandelt werden können. Das der vorliegenden Erfindung zugrunde liegende technische Problem besteht vor allem darin, ein verbessertes Verfahren zur Herstellung einer konditionierten Blutzusammensetzung, weiche bestimmte induzierte Faktoren oder Cytokine enthält und effektiv zur Behandlung und Vorbeugung eingesetzt werden kann. bereitzustellen.

Das zugrunde liegende technische Problem wird im Wesentlichen gelöst durch die Bereitstellung eines Verfahrens zur Herstellung einer konditionierten Blutzusammensetzung aus Blut, wobei das Verfahren zumindest folgende Schritte aufweist:

In Schritt (a) wird Blut, vorzugsweise venöses Vollblut, aus einem menschlichen oder tierischen Körper in an sich bekannter Weise, vorzugsweise frisch mittels Venenpunktion, entnommen. Im, vorzugsweise unmittelbar nachfolgenden Schritt (b) wird das entnommene Blut in mindestens einem modifizierten Gefäß inkubiert, um Faktoren oder Cytokine in der Blutzusammensetzung zu induzieren, das heißt die Produktion und Freisetzung solcher Faktoren oder Cytokine im Blutgewebe anzuregen. Erfindungsgemäß beträgt die Temperatur bei der Inkubation des Blutes in dem modifizierten Gefäß von 10 bis 40°C, bevorzugt von 25 bis 40 °C, weiter bevorzugt etwa 37 °C. Im Schritt (c) wird eine konditionierte Blutzusammensetzung, welche reich an bestimmten induzierten Faktoren oder Cytokinen ist, in dem modifizierten Gefäß erhalten.

Erfindungsgemäß wird bei der Inkubation des Blutes mindestens ein modifiziertes Gefäß verwendet, welches dadurch gekennzeichnet ist, dass es eine innere Oberfläche pro 1 ml inkubiertem Blut von mindestens etwa 100 mm²/ml oder mehr, besonders 104 mm²/ml oder mehr, 123 mm²/ml oder mehr, 131 mm²/ml oder mehr, 224 mm²/ml oder mehr oder 283 mm²/ml oder mehr, aufweist. In einer bevorzugten Variante weist das Gefäß eine innere Oberfläche von etwa 200 bis etwa 750 mm²/ml, besonders bevorzugt etwa 250 bis etwa 650 mm²/ml, auf.

Vorzugsweise weist das Gefäß ein Füllvolumen von 5 ml oder mehr, 10 ml oder mehr, 50 ml oder mehr, 60 ml oder mehr oder 100 ml oder mehr auf. Ist beispielsweise vorgesehen, eine Menge von etwa 50 ml Blut zu entnehmen und zu inkubieren, so soll die innere Oberfläche des modifizierten Gefäßes erfindungsgemäß mindestens etwa 6.600 mm² (66 cm²), vorzugsweise etwa 10.000 mm² bis etwa 37.500 mm² (100 bis 375 cm²) aufweisen. ist beispielsweise vorgesehen, eine Menge von etwa 10 ml Blut zu entnehmen und zu inkubieren, so soll die innere Oberfläche des modifizierten Gefäßes erfindungsgemäß mindestens etwa 2.300 mm² (23 cm²), vorzugsweise etwa 2.500 mm² bis etwa 7.500 mm² (25 bis 75 cm²) aufweisen.

Unter "innerer Oberfläche" des Gefäßes wird diejenige Oberfläche im Inneren des Gefäßes verstanden, die bei der Inkubation mit der zu konditionierenden Blutzusammensetzung in Kontakt steht, das heißt im Wesentlichen davon benetzt ist.

Die Erfindung sieht also vor, dass Blut, das einem menschlichen oder tierischen Körper entnommen wurde, in einem speziellen modifizierten Gefäß mit einem bestimmten Oberflächenindex der inneren Oberfläche von 200 mm²/ml oder mehr zu inkubieren. Die Erfinder fanden überraschend, dass durch das erfindungsgemäße Vorgehen bereits nach vergleichsweise kurzer Zeit eine konditionierte Blutzusammensetzung in dem modifizierten Gefäß erhalten werden kann, die einen hohen Anteil bestimmter induzierter Faktoren enthält und worin beispielsweise der Faktor IL-6 in hoher Konzentration vorliegt. Dabei führt das erfindungsgemäße Vorgehen überraschenderweise zu einer Blutzusammensetzung, die prophylaktisch und therapeutisch hohe Wirksamkeit besitzt. So können beispielsweise entzündliche Gelenkerkrankungen, Augenentzündung beim Pferd, Sehnenvedetzungen, Nervenverietzungen, Endometriose. Neurodermitis sowie Muskelverletzungen wirksam therapiert werden, indem die erfindungsgemäß erhaltene konditionierten Blutzusammensetzung als Blutserumzusammensetzung in den erkrankten Organismus oder in oder auf das erkrankte Organ verabreicht wird.

Durch das erfindungsgemäße Vorgehen kann beispielsweise eine konditionierte Blutzusammensetzung aus frisch entnommenem Vollblut menschlicher Spender erhalten werden, worin IL-6 in frisch entnommenem einem Anteil von mehr als 2000 pg/ml vorliegt. Im Vergleich dazu beträgt der Anteil an IL-6 in unkonditioniertem Vollblut von etwa 0,5 bis etwa 15 pg/ml. In der Regel wird erfindungsgemäß also eine etwa 200-fache bis etwa 4000-fache Steigerung des Gehalts an IL-6 erreicht

Neben dem besonders beachtenswerten Faktor IL-6 werden weitere therapeutisch und prophylaktisch wirksame Komponenten wie Faktoren oder Cytokine in der konditionierten Blutzusammensetzung in hohem Anteil erhalten. Dazu zählen neben den bekannten Faktoren IL-4, IL-10 und IL-1Ra überraschenderweise auch Faktoren wie Interleukin-13 (IL-13), Interleukin-1 (IL-1), vor allem IL-1β, Tumor necrosis factor (TNF), Insulin-like growth factor (IGF), Transforming growth factor (TGF), Platelet-derived growth factor (PDGF), Fibroblast growth factor (FGF) und Hepatocyte growth factor (HGF). Es liegt in der erfindungsgemäß herstellbaren konditionierten Blutzusammensetzung also vorteilhafterweise ein "Cocktail" verschiedener effektiv induzierter Faktoren oder Cytokine vor. Ohne an die Theorie gebunden zu sein, stellt gerade der erfindungsgemäß erhältliche "Cocktail" von Faktoren und Cytokinen die therapeutisch und prophylaktisch hochwirksame Wirkstoffzusammensetzung dar.

Dabei können die oben genannten Wirkstoffe innerhalb der Blutzusammensetzung auch in Form von Vesikeln, Mikrovesikeln oder Exosomen vorliegen. Unter Vesikeln bzw. Mikrovesikeln versteht man subzelluläre Bestandteile, die unter anderem von der Membranoberfläche von Immunzellen abgeschnürt werden können. Unter Exosomen sind subzelluläre Bestandteile zu verstehen, die vesikelförmige Strukturen im Nanometerbereich darstellen und durch Einstülpungen von sog. "multivesicular bodies" und Sekretion durch Immunzellen entstehen.

Unter einer "Blutzusammensetzung" wird vorliegend eine Zusammensetzung aus Blut, vor allem bestehend aus Blutplasma, Serum und Blutzellen, verstanden, die mindestens eine Komponente enthält, die ausgewählt ist aus Proteinen wie Faktoren und Cytokinen. Vorliegend wird unter einer Blutzusammensetzung auch eine Blutserumzusammensetzung verstanden. Eine Blutserumzusammensetzung unterscheidet sich von einer Blutzusammensetzung vor allem dadurch, dass die Blutserumzusammensetzung keine zellulären Bestandteile (mehr) enthält. Eine konditionierte Blutserumzusammensetzung wird aus einer erfindungsgemäß erhältlichen konditionierten Blutzusammensetzung beispielsweise dadurch erhalten, dass aus der Blutzusammensetzung die zellulären Bestandteile mittels Zentrifugation, Filtration oder anderen geeigneten Maßnahmen abgetrennt werden, sodass eine zellfreie Lösung aus Blutplasma und Serumbestandteilen, die zumindest die induzierten Faktoren und Cytokinen enthält, erhalten wird.

In einer bevorzugten Ausführung werden demgemäß in einem weiteren Schritt die zellulären Bestandteile aus der erhaltenen konditionierten Blutzusammensetzung vollständig oder im Wesentlichen vollständig abgetrennt, sodass eine konditionierte Blutserumzusammensetzung erhalten wird. Das konditionierte Blutserum kann wie die erfindungsgemäß erhältliche Blutzusammensetzung eingesetzt werden und vermittelt in der Regel dieselben erfindungsgemäßen technischen Vorteile. Der Fachmann wird je nach Anwendungsgebiet und Zweckmäßigkeit die konditionierte Blutserumzusammensetzung oder die konditionierte Vollblutzusammensetzung einsetzen. Vorzugsweise wird er die konditionierte Blutserumzusammensetzung einsetzen.

Bevorzugt wird die Inkubation des Blutes in dem mindestens einen modifizierten Gefäß solange durchgeführt, bis die Induktion der Faktoren oder Cytokine genügend weit vorangeschritten ist. Die induzierten Faktoren oder Cytokine werden vom Blutgewebe im Wesentlichen ab dem Zeitpunkt des Beginns der Inkubation an produziert und sezerniert, sodass eine wirksame Menge der induzierten Faktoren oder Cytokine in der konditionierten Blutzusammensetzung akkumuliert.

In einer Ausführung der Erfindung zeigt das Auftreten IL-6 in der Blutzusammensetzung die erfolgreiche und genügend weit fortgeschrittene Induktion an. Besonders beträgt der Anteil an IL-6 dabei mindestens 30 pg/ml. Vorzugsweise wird in dem modifizierten Gefäß solange inkubiert, bis zumindest 30 pg/ml IL-6 in der Blutzusammensetzung vorliegen. In weiter bevorzugten Varianten wird solange inkubiert, bis zumindest 200 pg/ml, bevorzugt 500 pg/ml, besonders bevorzugt 1000 pg/ml in der Blutzusammensetzung vorhanden sind.

In einer weiteren Ausführung wird für einen Zeitraum von 36 Stunden oder weniger inkubiert. In einer weiteren Ausführungsform wird für einen Zeitraum von 9 Stunden oder weniger inkubiert. In einer weiteren Variante wird für einen Zeitraum von 2 oder mehr und bis 36 oder weniger, vorzugsweise bis 9 oder weniger Stunden inkubiert.

In einer weiteren Ausführung findet die Inkubation des Blutes unter geringem Sauerstoffpartialdruck (pO₂) statt. Besonders beträgt der Sauerstoffpartialdruck während der Inkubation weniger als 5 kPa, vorzugsweise weniger als 3 kPa. In einer bevorzugten Variante findet die Inkubation des Blutes in dem modifizierten Gefäß unter Sauerstoffabschtuss statt.

In bevorzugter Ausführung weist das modifizierte Gefäß in seinem Inneren besondere Strukturen mit großer Oberfläche auf, sodass die sich aus der (äußeren) Geometrie des Gefäßes primär ergebende innere Oberfläche durch die besonderen Strukturen vergrößert wird. Die Cberflächenvergrößerung durch die besonderen Strukturen beträgt vorzugsweise von 10 % bis etwa 200 %, in einer Variante von 10 % bis 100 %. Dazu zählen bevorzugt Strukturen mit einem großen Oberfläche/Volumenverhältnis wie Kugeln und Fasern, aber auch andere Partikel wie Mehl und Granulat, oder Kombinationen solcher Strukturen. Die Oberfläche dieser Strukturen ist vorzugsweise glatt. Alternativ können in einigen Fällen Strukturen mit rauer Oberfläche eingesetzt werden.

Der Fachmann wird die Zahl und Form der inneren Strukturen nach Anwendungsgebiet und Zweckmäßigkeit wählen. Es versteht sich, dass die Form und Zahl der zuzugebenden inneren Strukturen dabei so gewählt wird, dass die Summe aus der Oberfläche der zugegebenen inneren Strukturen und der inneren Oberfläche des zu modifizierenden Gefäßes so aufeinander abgestimmt wird, dass das gemäß der Erfindung vorgesehene Oberflächen/Volumen-Verhältnis (Oberflächenindex) erhalten wird.

Bevorzugt weist das modifizierte Gefäß eine nicht pyrogene innere Oberfläche auf. Vorzugsweise ist das modifizierte Gefäß aus pyrogenfreiem Werkstoff aufgebaut.

Werden partikelförmige innere Strukturen wie Kugeln, Fasern, Mehl, Granulat oder Mischungen daraus eingesetzt, so enthalten diese oder bestehen vorzugsweise aus Werkstoffen ausgewählt aus Metallen, Metalloxiden oder Kunststoffen und Mischungen daraus. Bevorzugte Beispiele dafür sind Glas, Korund, Quarz, Polystyrol, Polyvinylchlorid, Polyethylen und Polypropylen sowie Mischungen daraus. Besonders bevorzugt ist Borosilikatglas. Diese Werkstoffe sind bevorzugt pyrogenfrei.

Vorzugsweise enthält das mindestens eine modifizierte Gefäß in seinem inneren Glaskugeln, besonders bevorzugt aus pyrogenfreiem Borosilikatglas, wobei die Glaskugeln einen (mittleren) Durchmesser von 0,5 bis 5 mm, bevorzugt 1,5 mm, 2,5 mm oder 3,5 mm, aufweisen. Besonders bevorzugt werden dem zu modifizierenden Gefäß, je nach Aufnahmekapazität des Gefäßes, die Glaskugeln in einer Zahl von etwa 10 bis 500 beigefügt. Ist beispielsweise ein Gefäß für die Aufnahme von etwa 50 ml Blut vorgesehen, sollen vorzugsweise etwa 30 bis 300 Kugeln, besonders etwa 50 bis 250 Kugeln eingefüllt werden, die einen Durchmesser von vorzugsweise 3,5 mm aufweisen.

In besonders bevorzugter Ausführung wird ein, vorzugsweise aus der Transfusionsmedizin bekanntes, Gefäß zur Blutentnahme beziehungsweise Blutaufbewahrung, wie Spritze, Blutröhrchen oder Blutbeutel, verwendet, das durch Zugabe eines bestimmten Anteils solcher innerer Strukturen modifiziert wird, sodass ein modifiziertes Gefäß mit einer vergrößerten inneren Oberfläche erhalten wird. Die Erfindung sieht demgemäß zur Herstellung einer konditionierten Blutzusammensetzung die Verwendung mindestens eines modifizierten Gefäßes mit großer innerer Oberfläche mit erfindungsgemäßem Oberflächenindex, welches innere Strukturen ausgewählt aus Kugeln, Fasern, Mehl, Granulat, Partikel oder Kombinationen daraus enthält, vor.

Der Fachmann kann selbstverständlich auch andere oder zusätzliche Maßnahmen treffen, um ein modifiziertes Gefäß mit vergrößerter innerer Oberfläche zu erhalten, das gemäß der Erfindung eingesetzt werden kann. In einer weiteren bevorzugten Ausführung ist ein Gefäß vorgesehen, dessen innere Gefäßwände Ausstülpungen, Kavitäten und/oder Vorsprünge aufweist, sodass das erfindungsgemäß vorgesehene Oberflächen/Volumen-Verhältnis (Oberflächenindex) erreicht wird.

In einer bevorzugten Ausführungsform weist das modifizierte Gefäß elastische Gefäßwände auf, die vorzugsweise eine luftfreie Entnahme von Blut aus dem tierischen oder menschlichen Körper ermöglichen, wenn sich das im Wesentlichen noch luftleere modifizierte Gefäß erst mit dem Einströmen des Bluts erweitert, sodass kein unerwünschter Luftraum im Gefäß entstehen kann. Es versteht sich, dass sich die Zahl und Oberfläche der in dem modifizierten Gefäß zur Vergrößerung seiner inneren Oberfläche vorgesehenen inneren Strukturen nicht an der maximalen Kapazität des elastischen Gefäßes orientiert, sondern vielmehr an dem Volumen der zu inkubierenden Blutzusammensetzung.

Vorzugsweise ist ein solches elastisches Gefäß ausgewählt aus in der Transfusionsmedizin vorgesehenen Blutbeuteln, welche vorzugsweise Einfach-, Zweifach-, Dreifach- oder Mehrfachbeutelsysteme sind. Während sich ein Einfachbeutelsystem dadurch auszeichnet, dass in der Regel zumindest eine Öffnung zum Befüllen beziehungsweise entleeren aufweist, stellen Zweifach-, Dreifach- und Mehrfachbeutel Anordnungen mehrere untereinander kommunizierender Beutel dar, die vorzugsweise mittels Schlauchverbindung miteinander in Kontakt stehen. Vorzugsweise sind solche Beutel in einfacher Weise aus zwei aufeinander verschweißten elastischen Folien aufgebaut.

In einer besonders bevorzugten Ausführungsform ist das mindestens eine erfindungsgemäß eingesetzte modifizierte Gefäß ein Blutbeutel oder Blutbeutelsystem, das durch Einfüllen einer gemäß der Erfindung gewählten Zahl und Art von Partikeln, vorzugsweise Glaskugeln, modifiziert wurde.

Vorzugsweise ist das Gefäß ein Beutelsystem, wie es als zweikammeriges Blutbeutelsystem für Zentrifugen zur Trennung von Blutbestandteilen in frisch entnommenen Blut verwendet wird. Ist das Gefäß gemäß der Erfindung, vorzugsweise durch Einfüllen von Glaskugeln, modifiziert, kann darin das erfindungsgemäße Verfahren zur Herstellung einer konditionerten Blutzusammensetzung durchgeführt werden. Anschließend werden aus dem konditionierten Blut die Blutkomponenten in dem Blutbeutelsystem aufgetrennt, das ein von "festen" Blutbestandteilen freie konditionierte Blutserumzusammensetzung erhalten wird.

Ein bevorzugtes zweikammeriges Blutbeutelsystem umfasst mindestens ein Primärgefäß und mindestens ein Sekundärgefäß, welche ein kommunizierendes Gefäßsystem bilden. Primärgefäß und Sekundärgefäß stehen über mindestens eine, insbesondere verschließbare, Überleitung in Verbindung. Im Zusammenhang mit der vorliegenden Erfindung wird unter einem "Primärgefäß" vorzugsweise ein Gefäß, das heißt Behälter, verstanden, worin die zu konditionierende und nachfolgend gegebenenfalls in ihre Einzelkomponenten aufzutrennende Blutzusammensetzung eingebracht wird, inkubiert wird und gegebenenfalls einer ersten Fraktionierung unterzogen wird. Besonders bevorzugt sind Primärgefäß, Sekundärgefäß und Überleitung zweckmäßigerweise an einer Trägerplatte fixiert. Besonders bevorzugt ist die Überleitung durch mindestens eine Unterbrechung welche als Ventil, Hahn und/oder Stopfen ausgebildet sein kann, verschließbar. Unter einem "Sekundärgefäß" wird ein vorzugsweise Gefäß, das heißt Behälter, verstanden, worin die gegebenenfalls vollständig oder teilweise im Primärgefäß in ihre Einzelkomponenten aufgetrennte Flüssigkeit oder Suspension vollständig oder teilweise eingebracht wird und einer zweiten Fraktionierung unterzogen wird. Jedes dieser Gefäße ist vorzugsweise mit jeweils mindestens einer, insbesondere verschließbaren, Ab- und/oder Zuleitung, insbesondere zur Zuführung, das heißt Einführen beziehungsweise Reapplikation, von Blutkomponenten und/oder Abführen, das heißt Entnehmen, von Blutkomponenten vorgesehen. Bevorzugt sind die erfindungsgemäßen zusätzlichen inneren Strukturen wie Glaskugeln im Primärgefäß vorgesehen beziehungsweise darin eingefüllt.

In einer bevorzugten Ausführungsform wird der Beutel beziehungsweise das Blutbeutelsystem für die Abtrennung der festen Blutbestandteile von der konditionierten Blutserumzusammensetzung mittels Zentrifugation in einen Zentrifugenbecher eingesetzt. Dieser ist bevorzugt so gestaltet, dass das bevorzugt als elastischer Beutel ausgeführte Gefäß bei der Zentrifugation so gedehnt wird, dass die Gefäßwände, teilweise und/oder vollständig an der Innenwand des Zentrifugenbechers zu liegen kommen. Bevorzugt ist die Benutzung eines sterilen Bechers. Besonders vorteilhaft werden so die Dehnungsbelastung der Gefäßwände und der enthaltenen Zellen während der Zentrifugation reduziert. Die bevorzugte Verwendung eines Zentrifugenbechers erlaubt auch den Einsatz von mechanisch leichterem, dünnerem und weniger stabilem Wandmaterial für den bevorzugten elastischen Beutel.

In einer bevorzugten Ausführung des Verfahrens ist die inkubierte Blutzusammensetzung eine allogene Blutzusammensetzung vorzugsweise eine Blutzusammensetzung, die in Form von Vollblut einem menschlichen oder tierischen Spender entnommen wird und nach Durchführung des erfindungsgemäßen Verfahrens als konditionierte Blutzusammensetzung, vorzugsweise als konditionierte Blutserumzusammensetzung einem menschlichen Spender verabreicht werden kann. In einer Variante ist die Blutzusammensetzung autolog, das heißt Spenderorganismus und Empfängerorganismus sind identisch. In dieser besonders bevorzugten Variante können alle Vorteile der autologen Spende zum Tragen kommen. Der Fachmann wird die Art und Identität des Spenders je nach Anwendung und Zweckmäßigkeit wählen. Dabei können im Allgemeinen die bekannten Kriterien und Vorteile, die für die Wahl einer autologen Spende relevant sind, in Betracht gezogen werden.

In einer alternativen Variante ist die Blutzusammensetzung xenogen. Das heißt, sie wird einem fremdartigen Organismus entnommen. Vorzugsweise wird dazu einem tierischen Spenderorganismus, beispielsweise einem Schwein, die unkonditionierte Blutzusammensetzung in Form von Vollblut entnommen und nach Durchführung des erfindungsgemäßen Verfahrens die konditionierte Blutzusammensetzung in das zu behandelnde Individuum, das einer anderen Art angehört, beispielsweise Pferd, Mensch oder Sportler, verabreicht.

Ein weiterer Aspekt der Erfindung ist die Bereitstellung einer konditionierten Blutzusammensetzung, welche durch das erfindungsgemäße Verfahren herstellbar ist oder bevorzugt hergestellt wird. Diese kann erfindungsgemäß zur Behandlung, Linderung, Heilung oder Vorbeugung eines Leidens des menschlichen oder tierischen Körpers eingesetzt werden. Erfindungsgemäß enthält diese Blutzusammensetzung die bei der Durchführung des erfindungsgemäßen Verfahrens induzierten Faktoren, mindestens 30, bevorzugt mehr als 200, 1000, 5000, 10 000 pg/ml, vorzugsweise von 30 bis 20 000 pg/ml Interleukin-6. Es versteht sich, dass die mittels des erfindungsgemäßen Verfahrens herstellbare konditionierte Blutzusammensetzung neben Interleukin-6 als einem der induzierten Faktoren weitere induzierte Faktoren aufweist. Es konnte überraschenderweise gezeigt werden, dass gerade die erfindungsgemäß erhältliche Zusammensetzung der induzierten Faktoren in einem "Cocktail" die erfindungsgemäßen Vorteile und Wirkungen aufweist.

Bevorzugt ist eine konditionierte Blutzusammensetzung, welche neben Interleukin-6 (IL-6) mindestens eine weitere Komponente aufweist, die ausgewählt ist aus: Interleukin-1 receptor antagonist (IL-1Ra), Interleukin-4 (IL-4), Interleukin-13 (IL-13), Interleukin-1 (IL-1), Interleukin-10 (IL-1), Tumor necrosis factor (TNF), Insulin-like growth factor (IGF), Transforming growth factor (TGF), Platelet-derived growth factor (PDGF), Fibroblast growth factor (FGF) und Hepatocyte growth factor (HGF).

In einer Variante enthält die konditionierte Blutzusammensetzung Interleukin-1 receptor antagonist (IL-1 Ra) in einem Anteil von 30 bis 50.000 pg/ml. In einer weiteren Variante enthält die konditionierte Blutzusammensetzung Interleukin-4 (IL-4) in einem Anteil von 2 bis 100 pg/ml. In einer weiteren Variante enthält die konditionierte Blutzusammensetzung Interleukin-13 (IL-13) in einem Anteil von 2 bis 100 pg/ml. In einer weiteren Variante enthält die konditionierte Blutzusammensetzung Interleukin-1 (IL-1) in einem Anteil von 5 bis 1.000 pg/l. In einer weiteren Variante enthält die konditionierte Blutzusammensetzung Interleukin-10 (IL-10) ein einem Anteil von 5 bis 1.000 pg/l. In einer weiteren Variante enthält die konditionierte Blutzusammensetzung Tumor necrosis factor (TNF) in einem Anteil von 5 bis 1.000 pg/ml. In einer weiteren Variante enthält die konditionierte Blutzusammensetzung Insulin-like growth factor (IGF) in einem Anteil von 100 bis 15.000 pg/ml. In einer weiteren Variante enhält die konditionierte Blutzusammensetzung Transforming growth factor (TGF) in einem Anteil von 10 bis 20.000 pg/ml. In einer weiteren Variante enthält die konditionierte Blutzusammensetzung Platelet-derived growth factor (PDGF) in einem Anteil von 100 bis 10.000 pg/ml. In einer weiteren Variante enthält die konditionierte Blutzusammensetzung Fibroblast growth factor (FGF) in einem Anteil von 50 bis 10.000 pg/ml. In einer weiteren Variante enthält die konditionierte Blutzusammensetzung Hepatocyte growth factor (HGF) in einem Anteil von 50 bis 10.000 pg/ml.

Überraschenderweise wirkt gerade der in der erfindungsgemäß erhältlichen konditionierten Blutzusammensetzung vorliegende Cocktail induzierter Faktoren und Cytokine prophylaktisch und therapeutisch besonders effektiv. Erfindungsgemäß wird mittels der konditionierten Blutzusammensetzung beziehungsweise der daraus erhältlichen konditionierten Blutserumzusammensetzung besonders wirkungsvoll bei einer Reihe von Krankheiten beziehungsweise Leiden des menschlichen oder tierischen Körpers eingesetzt. die damit behandelt, geheilt oder gelindert werden beziehungsweise womit gegen diese Krankheiten und Leiden vorgebeugt wird.

Erfindungsgemäß wird die erfindungsgemäß erhältliche konditionierte Blutzusammensetzung beziehungsweise Blutserumzusammensetzung bei Muskelerkrankungen, bei Erkrankungen des Stütz- und Bewegungsapparats wie auch Entzündungen und Reizungen des Nervensystems, vor allem Erkrankungen des Sehnenapparates wie Sehnenverletzungen, Sehnenscheidenentzündung, Bänderverletzungen, Sehnendegeneration und Bänderdegeneration, sowie zur schnellen Heilung. Linderung beziehungsweise zur Vorbeugung von Allergien, Unverträglichkeiten gegenüber Nahrungsmitteln oder Arzneimitteln, Erkrankungen mit Beteiligung des Immunsystems. vor allem Autoimmunerkrankungen, besonders des rheumatoiden Formenkreises sowie Erkrankungen bedingt durch Neurodermitis, und zur Behandlung und Heilung chronischer Wunden, besonders diabetischer Ulcera, die Behandlung von Endometriose, sowie die Behandlung von chronischer Augenentzündung und die Regeneration beziehungsweise Schmerzbesserung von Reizungen der Sehnen bei Pferd, eingesetzt. Zu den Muskelerkrankungen zählen Muskelerkrankungen, die durch Muskelverletzungen bei Muskeloperationen, im Zusammenhang mit Muskelfaserrissen bei Muskeldegenerationen, bei Muskeldefekten, bei Muskelatrophie, bei Muskelbruch. bei Muskeldystrophie entstehen, beziehungsweise auf Muskelermüdung oder Muskelkater zurückzuführen sind.

Die vorliegende Erfindung betrifft daher die Verwendung der erfindungsgemäßen Blutzusammensetzung der daraus erhältlichen konditionierten Blutserumzusammensetzung zur Behandlung oder Vorbeugung eines Leidens des menschlichen oder tierischen Körpers. Das Leiden des menschlichen oder tierischen Körpers. das bevorzugt behandelt, gelindert oder geheilt oder wogegen vorgebeugt werden kann, ist ausgewählt aus Krankheiten des rheumatoiden Formenkreises, Krankheiten des Stütz- und Bewegungsapparates sowie Krankheiten, die im Zusammenhang mit dem Immunsystem stehen sowie Krankheiten, die akute oder chronische Schmerzen verursachen.

Es versteht sich, dass zur adäquaten Behandlung der jeweiligen Erkrankung der Fachmann die jeweils zweckmäßige Applikationsart für die Verabreichung der erfindungsgemäßen konditionierte Blutzusammensetzung wählt. Vorzugsweise wird die konditionierte Blutzusammensetzung oder Blutserumzusammensetzung in den Körper und/oder das betroffene Organ wie Gelenk, Muskel, Sehnen,Haut oder Nerv injiziert oder infundiert, und zwar bevorzugt intravenös, intraarteriell, subkutan, intradermal, subkonjunktival, topikal, intrathekal, perispinal, in und/oder an zentrale Nerven, in und/oder an periphere Nerven, intraartikulär und/oder intramuskulär.

In einem weiteren Aspekt der Erfindung wird die erfindungsgemäße konditionierte Blutzusammensetzung daher zur Herstellung eines Arzneimittels zur Behandlung oder Vorbeugung eines Leidens des menschlichen oder tierischen Körpers verwendet. Diese Leiden sind vorstehend charakterisiert.

Daneben ist eine Verwendung der erfindungsgemäßen Blutzusammensetzung auch als nicht therapeutisches Kosmetikum, als sogenanntes "anti-aging"-Mittel, vorgesehen. Es hat sich gezeigt, dass durch systematische und/oder topische Verabreichung körperliche Begleiterscheinungen des Alters, vor allem die vorgenannten Symptome, gelindert der geheilt aber auch das äußere Erscheinungsbild: Haut, Haare, Nägel verbessert werden kann. In einem weiteren Aspekt der Erfindung wird die erfindungsgemäße Blutzusammensetzung zur Herstellung von Kosmetika verwendet.

Schließlich betrifft die vorliegende Erfindung auch ein Verfahren zur Behandlung oder Vorbeugung eines vorstehend charakterisierten Leidens des menschlichen oder tierischen Körpers, welches zumindest folgenden Schritt enthält: Verabreichung der erfindungsgemäß konditionierten Blutzusammensetzung in den menschlichen oder tierischen Körper in einer therapeutisch oder prophylaktisch wirksamen Dosis. Dosis und Applikationsart wählt der Fachmann nach Anwendungsgebiet und Zweckmäßigkeit.

### Ausführungsbeispiele

Die Erfindung wird durch die nachfolgenden Beispiele und die Figur näher erläutert, wobei die Beispiele nicht beschränkend zu verstehen sind. Der Fachmann wird aus den Beispielen das Grundprinzip der Erfindung und die damit verbundenen technischen Vorteile erkennen. Er wird in der Lage sein, die Grundprinzipien und technischen Vorteile auf andere, hier nicht ausdrücklich angesprochene Bereiche zu übertragen.

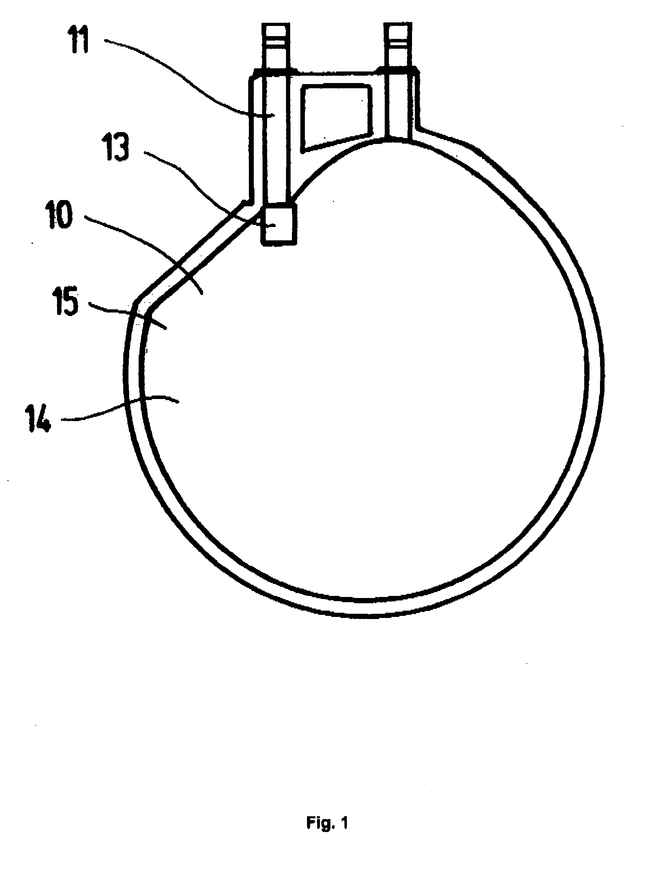

Figur 1 zeigt eine schematische Darstellung einer bevorzugten Ausführungsform der erfindungsgemäßen Vorrichtung, bestehend aus einem als elastischen Beutel ausgebildeten Gefäß (10) mit einem bevorzugt halbkreisförmigen unteren Abschnitt (14) und einen sich vorzugsweise verjüngenden oberen Abschnitt (15) mit mindestens einer Zu- und/oder Ableitung (11), welche in den trichterförmigen oberen Abschnitt (15) des Gefäßes mündet. An ihrem unteren Ende, welcher in das Lumen des Gefäßes 10 mündet, ist vorzugsweise ein Lippenventil (13), das heißt Flatterventil, vorgesehen.

### Beispiel 1: Kit zur Gewinnung von konditionierter Blutzusammensetzung aus Vollblut

Es wird ein sterilisierbarer Kit zum Einmalgebrauch zusammengestellt, das folgendes enthält:
- Ein Beutelsystem zur Inkubation des Bluts und zur Abtrennung von festen Blutbestandteilen (Figuren 1 und 2, Tabelle 1), beschickt mit Borosilikatglaskugeln (cirka 200 Stück) mit einem Durchmesser von 3,5 mm,
- 20-Gauge-Kanüle zum Aufziehen von Gerinnungshemmer in die Blutabnahmespritze.
- 60-ml-Spritze zur Blutabnahme,
- Butterfly-Kanüle zur Blutabnahme,
- 60-ml-Spritze zur Aufnahme der konditionierten Bluserumzusammensetzung

Alle Komponenten sind Einmalgebrauchsartikel, verpackt und gamma-sterilisiert und als Ganzes mit sterlier Umverpackung versehen.

Die Tabellen 1 und 2 listen die Materialien der verwendeten Komponenten auf.

**Tabelle 1:**

| Komponente | Material, Lieferant |
|---|---|
| Beutel (10) | Beutelfolie: PVC compound 3222 (Solvay Draka) |
| innere Strukturen | 200 Borosilikatglaskugeln, 3,5 mm Durchmesser (Duran®) |

**Tabelle 2: Kit zur Herstellung einer konditionierten Blutzusammensetzung**

| Komponente | Material, Lieferant | |
|---|---|---|
| erfindungsgemäße Vorrichtung | (siehe Tabelle 1) | |
| Butterfly-Kanüle 1,1 × 19 mm | Verschlusskappe: | PE |
| | LL-Adapter: | ABS transparent |
| | Flügel-Anschlusskopf: | PVC |
| | Schlauch: | PVC 60 Sh A |
| | Kanüle: | ISO 638/13 |
| | Schutzschlauch: | PE |
| Kanüle 1,1 x 40 mm | Anschlusskopf: | PP |
| | Schutzkappe: | PP |
| | Kanüle: | Edelstahl gemäß |
| | | DIN EN ISO 9626 |
| 60 ml Spritze | Zylinder: | PP |
| | Kolbenstange: | PP |
| | Kolbenstopfen: | Naturkautschuk |
| 10 ml Spritze (12 cc) | Zylinder: | PP |
| | Kolbenstange: | PP |
| | Kolbenstopfen: | PP |
| Perfusorleitung 1,5 m, 1,0 x 2,7 mm | LL male: | ABS KR 2802 |
| | Kappe: | PE, opak |
| | LL female: | PVC |
| | Kappe: | ABS. rot |
| | Schlauch: | |
| | inner Layer: | ND PE |
| | middle layer: | EVA |
| | outer layer: | PVC |
| Blisterverpackung | PET-GAG | |
| 0.9×206×500 mm | | |
| Tyvek-Siegelpapier | Tyvek 10MP/1073B | |

### Beispiel 2: Gewinnung einer konditionierten Blutserumzusammensetzuna aus Vollblut

### a) Blutabnahme

Die Blutabnahme erfolgt mit einer 60 ml Luerlock-Spritze. Die Spritze wird langsam bis zur 60 ml-Markierung mit Vollblut gefüllt. Es wird auf blasenfreie Füllung geachtet, damit sich tatsächlich genau 60 ml in der Spritze befinden.

### b) Befüllen des Beutelsystems und Inkubation

Der Inhalt der Spritze wird langsam und vollständig über die Zu-/Ableitung (11) in das als elastischer Beutel ausgebildete Gefäß (10) eingefüllt. Im Gefäß sind cirka 200 Kugeln aus Borosilikatglas (Duran®), Durchmesser 3,5 mm, vorgelegt.

Die Spritze wird nach der Befüllung abgeschraubt und der Anschluss (12) des Beutels wird mit einer neuen Verschlusskappe wieder verschlossen.

Das Gefäß (10) wird bei etwa 37°C für 9 bis 36 Stunden vorzugsweise hängend gelagert. Dabei wird das entnommene Blut im Gefäß mit den die innere Oberfläche vergrößernde Kugeln inkubiert. Die vergrößerte innere Oberfläche beträgt etwa 350 mm² pro 1 ml inkubiertem Blut.

### c) Abtrennen der festen Blutbestandteile

In ein steriles Zentrifugengehänge, in einen Zentrifugenbecher wird das Gefäß (10) eingesetzt. Nach Kontrolle des korrekten Gewichtsausgleiches wird die Zentrifugation bei etwa 2500 U/min für etwa 3 min durchgeführt. Nach Ablauf der Zentrifugation, in der eine Separation der zellulären Blutbestandteile von den flüssigen stattfindet, wird der Zentrifugenbecher zusammen mit dem Gefäß (10) vorsichtig entnommen.

Durch die Zentrifugation haben sich Blutzellen, hauptsächlich Erythrocyten (EZ) im unteren Abschnitt des Gefäßes (10) angesammelt. Die Zentrifugation trennt Serum vom Blutkoagel. Das Serum wird in den zweiten Beutel transferiert und anschließend gegebenenfalls ein zweites Mal zentrifugiert. Über den Entnahmeanschluss der Zu-/Ableitung (11) wird die konditionierte Serumzusammensetzung entnommen. Die gefüllte Spritze wird anschließend abgeschraubt.

### Beispiel 3: Analyse der konditionierten Blutserumszusammensetzung

Es wurden vier Versuchsansätze A, B, C, D und E hergestellt, die in gleicher Weise zur Inkubation von Vollblut verwendet wurden.

In einem Ansatz A wurde ein kommerziell erhälticher Blutbeutel (OSTEOKIN, Orthogen, Düsseldorf), der im Wesentlichen in den Beispielen 1 und 2 beschrieben ist, mit 210 Kugeln aus Borosilikatglas (Duran®) mit einem Durchmesser von 3,5 mm befüllt. Durch den Zusatz der inneren Strukturen beträgt die innere Oberfläche des modifizierten Gefäßes insgesamt etwa 18.125 mm². Bei Inkubation von 50 ml Blut beträgt das Oberflächen/VolumenVerhältnis (Oberflächenindex) des modifizierten Gefäßes etwa 360 mm²/ml.

In einem weiteren Ansatz B wurde dasselbe Blutbeutelsystem wie in Ansatz A eingesetzt, worin keine zusätzlichen inneren Strukturen eingefüllt wurden. Das unbefüllte Blutbeutelsystem weist eine innere Oberfläche von etwa 10.000 mm² auf. Bei 50 ml Blut entspricht dies einem Oberflächenindex von etwa 200 mm²/ml.

In einem weiteren Ansatz C wurde dasselbe Blutbeutelsystem wie In Ansatz A eingesetzt und mit 780 Glaskugeln mit einem Durchmesser von 3,5 mm befüllt. Die innere Oberfläche beträgt dann insgesamt etwa 40.000 mm². Bei einer Füllmenge von 50 ml Blut ergibt sich ein Oberflächenindex von etwa 800 mm²/ml.

In einem weiteren Ansatz D wurde ein anderes Blutentnahmesystem. das eine im Wesentlichen zylindrische Form aufweist, mit 36 Glaskugeln mit einem Durchmesser von 1,5 mm befüllt. Die innere Oberfläche beträgt dann insgesamt etwa 4050 mm². Bei einer Füllmenge von 10 ml Blut erglbt sich ein Oberflächenindex von etwa 405 mm²/ml.

In einem weiteren Ansatz E wurde ein Blutentnahmesystem, das eine im Wesentlichen zylindrische Form aufweist, mit 62 Glaskugeln mit einem Durchmesser von 3.5 mm befüllt. Die innere Oberfläche beträgt dann insgesamt etwa 6200 mm². Bei einer Füllmenge von 10 ml Blut ergibt sich ein Oberflächenindex von etwa 620 mm²/ml.

In allen Versuchsansätzen wurde jeweils venöses Vollblut frisch entnommen und jeweils in die Gefäße der Ansätze A, B und C eingefüllt. Die Gefäße wurden bei etwa 37°C für 24 Stunden (t = 24 h) inkubiert. Zusätzlich wurde als Kontrolle jeweils etwa 10 ml frisches Vollblut derselben Spender unmittelbar nach der Blutentnahme aufgearbeitet (t = 0 h).

Nach Verstreichen der Inkubationszeit wurden die Blutkomponenten IL-1Ra, IL-6, TNFa und IL-1β in den Blutzusammensetzungen quantifiziert.

Ergebnisse: Tabelle 3 zeigt die Ergebnisse.

**Tabelle 3:**

| Faktor/Cytokin [pg/ml] | t = 0 h | t = 24 h | | | | |
|---|---|---|---|---|---|---|
| | | A 360 mm²/ml | B 200 mm²/ml | C 800 mm²/ml | D 405 mm²/ml | E 620 mm²/ml |
| IL-1Ra | 323,7 | 8592 | 6626 | -* | 2663 | 7836 |
| IL-6 | 3,7 | 2830 | 1571 | -* | 847,5 | 2933 |
| TNFa | 19.9 | 718.3 | 204,5 | -* | 31.54 | 569.8 |
| IL-1β | 1,00 | 396,6 | 92.69 | -* | 16,81 | 154,8 |

| | | | | | | |
|---|---|---|---|---|---|---|
| *) Zelllyse, keine Messung | | | | | | |

Während die Ansätze A und B eine deutliche Induktion der analysierten Faktoren in der Blutzusammensetzung zeigten, trat bei Ansatz C während der Inkubation Hämolyse auf. Es zeigt sich, dass die Stärke der Induktion vom Oberflächenindex abhängt; bei größerem Oberflächenindex (größere innere Oberfläche) wird ein größerer Anteil induzierter Cytokine erhalten. Gleichzeitig existiert eine obere Grenze des Oberflächenindex; wird ein kritischer Wert überschritten, kommt es zur Hämolyse. Eine hämolysierte Blutzusammensetzung kann nicht weiterverwendet werden. Bei großen Oberflächenindices nahe am kritischen Wert, kann die Hämolyse in bestimmten Grenzen unterdrückt werden, indem die Inkubationszeit von 24 Stunden auf 6 bis 9 Stunden verkürzt wird (Daten nicht dargestellt).

### Beispiel 4: Cytokinprofil der Konditionietten_Blutzusammensetzung

In einem weiteren Ansatz wurden in ein zylindrisches Blutentnahmegefäß zur Vergrößerung der inneren Oberfläche 36 Glaskugeln aus Borosilikatglas (Ouran®) mit einem Durchmesser von 1.5 mm eingefüllt. Es wurden 50 ml frisch entnommenes Vollblut inkubiert. Der Oberflächenindex betrug etwa 405 mm²/ml.

In dem Blutentnahmegefäß wurde Blut bei etwa 37°C für drei Stunden, neun Stunden und 24 Stunden inkubiert. Anschließend wurde der Gehalt an den Cytokinen FGF, IL-4. IL-10, IL-1β, TNF, IL-6, IL-1Ra und TGFβ bestimmt.

### Ergebnisse:

Nach bereits drei Stunden Inkubation zeigt sich ein deutlicher Anstieg des Cytokingehalts in der konditionierten Blutzusammensetzung. In Tabelle 4 sind die nach 24 Stunden (t = 24 h) gemessenen Werte den unmittelbar nach der Blutentnahme gemessenen Werten (t = 0 h) gegenübergestellt.

**Tabelle 4**

| Faktor/Cytokin [pg/ml] | t = 0 h | t = 24 h |
|---|---|---|
| FGF | 0.1 | 2.0 |
| IL-4 | 5,4 | 7,9 |
| IL-10 | 7,9 | 55,4 |
| IL-1β | 3.9 | 409 |
| TNF | 6,0 | 536 |
| IL-6 | n.a. | 3444 |
| IL-1Ra | 241,9 | 9975 |
| TGFβ | 18313 | 36696 |

### Beispiel 5: Behandlung von Neurodermitis

Zur Behandlung von Neurodermitis wurde die erfindungsgemäß hergestellte konditionierte Blutzusammensetzung Patienten in Form von Injektionen, auch als intraartikuläre Injektionen, appliziert. Dabei werden 2 ml der konditionierten Blutzusammensetzung über einen Zeitraum von 3 Wochen im Abstand von jeweils 2 bis 3 Tagen injiziert. Eine Verbesserung der Symptome der Neurodermitis konnte innerhalb von 3 Tagen festgestellt werden, Ein. erneutes Aufflammen der Erkrankung nach ungefähr 2,5 Monaten wurde ebenfalls mit 3 Injektionen erfolgreich behandelt.

Bei anderen Patienten, bei denen intraartikuläre Injektionen, primär zur Behandlung ihrer Knieschmerzen (bedingt durch Arthrose und Beschwerden im Meniskus) eingesetzt wurden, besserten sich im Laufe von 6 Injektionen auch die Neurodermitissymptome. Seitdem ist es nicht mehr zu einem Aufflammen der Neurodermitis gekommen.

### Beispiel 6: Behandlung von Entzündung oder Irritationen des Nervensystems

In dieser Anwendung der erfindungsgemäß hergestellten konditionierten Blutzusammensetzung wurden Rückenschmerzpatienten (n = 30), die seit mindestens 6 Monaten chronisch an radikulär bedingten Rückenschmerzen litten, mit lokalen Injektionen an der Nervenwurzel (epidural-peridurale Injektion gem. Krämer et al.) behandelt. Die Schmerzen besserten sich innerhalb weniger Wochen, und der Effekt war im Mittel noch nach sechs Monaten manifest. Dabei stellte sich ein zumindest gleichwertiges oder leicht verbessertes Ergebnis im Vergleich zu Patienten ein, die mit gleicher Injektionstechnik mit entweder 5 mg oder 10 mg Glucocorticoid (Triamcinolon) als Vergleichssubstanz behandelt wurden.

### Beispiel 7: Behandlung von Endometriose

Patientinnen (n = 4), die an schmerzhafter Endometriose litten, wurden durch eine intraperitoneale Injektion von 4 ml des erfindungsgemäß hergestellten konditionierten Blutserums direkt in das durch die Endometriose hervorgerufene Geschwulstgewebe und/oder in den Bauchraum behandelt. Nach diesen zunächst mit heftigen Schmerzen verbundenen Applikationen, trat innerhalb von wenigen Stunden eine deutliche Reduktion der Schmerzen ein. Dieser Effekt setzte sich weiter fort und führte zu fast vollständiger Schmerzfreiheit am Folgetag. Als Fortführung der Therapie wurde in wöchentlichem Abstand mit subkutanen Injektionen von jeweils 2 ml des konditionierten Blutserum weiterbehandelt. Ein Rückfall beziehungsweise das erneute Auftreten von Schmerzen konnte bisher noch nicht beobachtet werden. Die schmerzhemmende Wirkung der erfindungsgemäß konditionierten Blutzusammensetzung geht überraschenderweise weit über die Wirkung normaler Schmerzmittel hinaus.

### Beispiel 8: Chronische Augenentzündung beim Pferd

Zur Behandlung der chronischen Augenentzündung beim Pferd (equine rezidivierende Uveitis, ERU) wurde die erfindungsgemäß hergestellte konditionierte Blutzusammensetzung 6 Pferden, von denen jeweils 3 Pferde in zwei unterschiedlichen Veterinärpraxen behandelt wurden, ins Auge (subkonjunktival) injiziert beziehungsweise ins Auge getropft (topical). Innerhalb des Nachuntersuchungszeitraums von bis zu 10 Monaten konnte bei keinem der behandelten Fälle ein Rückfall festgestellt werden.

### Beispiel 9: Regeneration und Schmerzbesserung von Reizungen_der_Sehnen beim Pferd

In einer weiteren Anwendung der konditionierten Blutzusammensetzung wurden Pferde mit Lahmheit, bedingt durch massive Entzündung beziehungsweise Reizung der Sehnenscheide wurden einhergehend mit Ergussbildung in der Sehnenscheide, mit Injektionen von je 3 ml der erfindungsgemäßen konditioniertem Blutserum in die Sehnenscheide behandelt. Dazu wurde zunächst in einem ersten Schritt der Erguss abpunktiert, um den Druck auf das Gewebe zu reduzieren und um proinflammatorische Substanzen zu entfernen. Nach der ersten Injektion reduzierte sich innerhalb einer Woche sowohl Lahmheit als auch die in der zweiten Woche feststellbare Ergussmenge deutlich. Nach 4 Wochen. also einer Woche nach Injektion der dritten und letzten Dosis in die Sehnenscheide, war eine fast vollständige Remission sowohl der Lahmheit als auch des Ergusses festzustellen.

Gleichartige Injektionen in sogenannte "core lesions" und/oder "superficial lesions" also degenerative Veränderungen innerhalb des Sehnengewebes, führten ebenfalls zu einer deutlichen Remission dieser klinischen Symptomatik. In einigen Fällen wurden Wiederauffüllungen des Defektes mit Collagenfasern beobachtet.

### Beispiel10: Behandlung von Wunden beim Pferd

Ein 14-jähriger an mehreren Gelenken lahmender Wallach litt seit vielen Wochen an einer persistierenden Wunde oberhalb des linken Vorderhufes des Hufes. Die erfindungsgemäße konditionierte Blutzusammensetzung wurde auf diese Wunde getropft (3 Tropfen auf eine Fläche von ca. 1x3 cm). Anschließend wurde die Wunde verbunden. Nach der abschließenden Begutachtung (nach drei Behandlungen in wöChentlichem Abstand) nach einem Zeitraum von 4 Wochen zeigte sich, dass sich die offene Wundfläche um ca. ein Drittel reduziert hatte.

## Patentansprüche

1. Verfahren zur Herstellung einer konditionierten Blutzusammensetzung, aus Blut, enthaltend die Schritte:
(a) Entnahme von Blut aus einem menschlichen oder tierischen Körper,
(b) Inkubieren des Blutes in einem modifizierten Gefäß mit innerer Oberfläche bei einer Temperatur von 10 bis 40 °C zur Konditionierung des Bluts, wobei Faktoren induziert werden, und wobei das modifizierte Gefäß eine innere Oberfläche von 104 mm² bis 750 mm² pro 1 ml inkubiertem Blut aufweist; und
(c) Erhalten einer konditionierten Blutzusammensetzung mit induzierten Faktoren in dem modifizierten Gefäß.

2. Verfahren nach Anspruch 1, wobei das Verfahren ein oder mehrere der folgenden Charakteristika aufweist:
(i) das Auftreten von Interleukin-6 (IL-6) in der Blutzusammensetzung in einem Anteil von mindestens 30 pg pro 1 ml zeigt die erfolgreiche Induktion an;
(ii) es wird für einen Zeitraum von 2 bis 36 Stunden inkubiert;
(iii) der Sauerstoffpartialdruck (pO₂) während der Inkubation beträgt weniger als 5 kPa:
(iv) in einem weiteren Schritt werden zelluläre Bestandteile aus der konditionierten Blutzusammensetzung abgetrennt und es wird eine konditionierte Blutserumzusammensetzung erhalten;
(v) das modifizierte Gefäß weist innere Strukturen mit großer Oberfläche auf, die ausgewählt sind aus Kugeln, Fasern, Mehl, Granulat, Partikel und Kombinationen davon, wobei die inneren Strukturen vorzugsweise mindestens einen Werkstoff ausgewählt aus Metall, Metalloxid und Kunststoff wie Glas, Korund, Quarz, Polystyrol, Polyvinylchlorid, Polyethylen, Polypropylen, oder Kombinationen davon enthalten;
(vi) das modifizierte Gefäß weist in seinem Inneren Glaskugeln auf, die einen Durchmesser von 0,5 bis 5 mm besitzen;
(vii) das modifizierte Gefäß weist elastische Gefäßwände zur luftfreien Entnahme von Blut aus dem tierischen oder menschlichen Körper auf, wobei das Gefäß vorzugsweise ausgewählt ist aus Blutbeuteln für die Transfusionsmedizin, insbesondere ausgewählt aus Einfach-, Zweifach-, Dreifach- und Mehrfachbeutelsystemen, und/oder wobei die elastischen Gefäßwände eine geringe Sauerstoffpermeabilität aufweisen;
(viii)die Blutzusammensetzung ist allogen:
(ix) die Blutzusammensetzung ist autolog;
(x) die Blutzusammensetzung ist xenogen.

3. Blutzusammensetzung, herstellbar durch das Verfahren nach einem der vorstehenden Ansprüche, zur Behandlung oder Vorbeugung eines Leidens des menschlichen oder tierischen Körpers, enthaltend 30 bis 20.000 pg/ml Interleukin-6 (lL-6)

4. Blutzusammensetzung nach Anspruch 3, enthaltend mindestens eine weitere Komponente ausgewählt aus:
- Interleukin-1 receptor antagonist (IL-1Ra),
- Interleukin-4 (IL-4),
- Interleukin-13 (IL-13),
- Interleukin-1 (IL-1),
- Interleukin-10 (IL-10),
- Tumor necrosis factor (TNF),
- Insulin-like growth factor (IGF),
- Transforming growth factor (TGF),
- Platelet-derived growth factor (PDGF),
- Fibroblast growth factor (FGF) und
- Hepatocyte growth factor (HGF).

5. Blutzusammensetzung nach Anspruch 3 oder 4, weiter enthaltend mindestens eine Komponente ausgewählt aus Vesikel, Mikrovesikel, Exosomen, *iRNA* und Gemischen davon.

6. Blutzusammensetzung nach Anspruch 3 bis 5, wobei die Blutzusammensetzung ein oder mehrere der folgenden Charakteristika aufweist:
(i) Interleukin-1 receptor antagonist (IL-1Ra) ist in einer Menge von 30 - 50.000 pg/ml vorhanden;
(ii) lnterleukinr-4 (IL-4) ist in einer Menge von 2-100 pg/ml vorhanden;
(iii) Interleukin-13 (IL-13) ist in einer Menge von 2-100 pg/ml vorhanden;
(iv) Interleukin-1 (IL-1) ist in einer Menge von 5 -1.000 pg/ml vorhanden;
(v) Interleukin 10 (IL-10) ist in einer Menge von 5 - 1.000 pg/ml vorhanden;
(vi) Tumor necrosis factor (TNF) ist in einer Menge von 5 -1.000 pg/ml vorhanden;
(vii) Insulin-like growth factor (IGF) ist in einer Menge von 100 -15-000 pg/ml vorhanden;
(viii)Transforming growth factor (TGF) ist in einer Menge von 10 - 20.000 pg/ml vorhanden;
(ix) Platelet-derived growth factor (PDGF) ist in einer Menge von 100 - 10.000 pg/ml vorhanden;
(x) Fibroblast growth factor (FGF) ist in einer Menge von 50 - 10.000 pg/ml vorhanden;
(xi) Hepatocyte growth factor (HGF) ist in einer Menge von 10 -10.000 pg/ml vorhanden.

7. Verwendung der Blutzusammensetzung nach einem der Ansprüche 3 bis 6 zur Behandlung oder Vorbeugung eines Leidens des menschlichen oder tierischen Körpers ausgewählt aus:
- Muskelerkrankungen,
- Erkrankungen des Sehnenapparates
- Allergien,
- Unverträglichkeiten gegenüber Nahrungsmitteln,
- Unverträglichkeiten gegenüber Arzneimitteln,
- Erkrankungen mit Beteiligung des Immunsystems
- Psoriasis und
- chronische Wunden wie diabetische Ulcera.

8. Verwendung nach Anspruch 7, wobei die Muskelerkrankung eine Muskelverletzung, eine Muskeloperation, ein Muskelfaserriss, eine Muskeldegeneration, ein Muskeldefekt, eine Muskelatrophie, ein Muskelbruch, eine Muskeldystrophie, eine Muskelermüdung oder Muskelkater ist.

9. Verwendung nach einem der Ansprüche 7 oder 8, wobei die Behandlung einer Muskelerkrankung eine Regeneration von Muskelgewebe umfasst.

10. Verwendung der Blutzusammensetzung nach einem der Ansprüche 3 bis 6 zur Behandlung oder Vorbeugung eines Leidens des menschlichen oder tierischen Körpers, ausgewählt aus:
- Neurodermitis,
- Entzündungen und Reizungen des Nervensystems,
- Endometriose,
- chronische Augenentzündung beim Pferd.

11. Verwendung nach einem der Ansprüche 7 bis 10, wobei die Blutzusammensetzung, gegebenenfalls zusammen mit pharmazeutischen Hilfsstoffen in den Körper oder das betroffene Organ injiziert wird.

12. Verwendung der Blutzusammensetzung nach einem der Ansprüche 3 bis 6 zur Herstellung eines Arzneimittels zur Behandlung oder Vorbeugung eines in den Ansprüchen 7 bis 10 charakterisierten Leidens des menschlichen oder tierischen Körpers.

13. Verwendung der Blutzusammensetzung nach einem der Ansprüche 3 bis 6 als Kosmetikum.
